# EUROPÄISCHE PATENTSCHRIFT

(11) **EP 0 910 292 B1**
(45) Veröffentlichungstag und Bekanntmachung des Hinweises auf die Patenterteilung: **20.07.2005**
(21) Anmeldenummer: 98902939.2
(22) Anmeldetag: 07.01.1998
(51) Int. Cl.: A61B 17/22, B06B 3/00

(54) **HANDSTÜCK FÜR EIN MULTIFUNKTIONALES ENDOSKOPISCHES OPERATIONSGERÄT SOWIE EIN DERARTIGES OPERATIONSGERÄT**
HANDPIECE FOR USE WITH A MULTIFUNCTIONAL OPERATING ENDOSCOPIC INSTRUMENT
PIECE A MAIN POUR APPAREIL OPERATOIRE ENDOSCOPIQUE MULTIFONCTIONNEL, ET L'APPAREIL OPERATOIRE

(30) Priorität: 07.01.1997 DE 19700270
(43) Veröffentlichungstag der Anmeldung: 28.04.1999
(73) Patentinhaber: Storz Endoskop GmbH, 8200 Schaffhausen (CH)
(72) Erfinder: NOVAK, P., CH-8200 Schaffhausen (CH); KRATTIGER, Beat, CH-8200 Schaffhausen (CH); IRION, Klaus, D-78532 Tuttlingen (DE); GMINDER, Frank, D-78532 Tuttlingen (DE); HENES, Rudolf, CH-8200 Schaffhausen (CH)
(74) Vertreter: Lohr, Georg, Dr.
(86) Internationale Anmeldenummer: PCT/DE1998/000041
(87) Internationale Veröffentlichungsnummer: WO 1998/030155

(56) Entgegenhaltungen:
- WO-A-93/16646
- WO-A-95/10233
- US-A- 3 433 226
- US-A- 5 507 738

## Beschreibung

### Technisches Gebiet

Die Erfindung bezieht sich auf ein Handstück für ein multifunktionales endoskopisches Operationsgerät, gemäß dem Oberbegriff des Patentanspruchs.

### Stand der Technik

Bei der Formulierung des Oberbegriffs des Patentanspruchs 1 wird von der US-PS 5,391,144, in der verschiedene Ultraschallbehandlungsvorrichtungen offenbart sind, ausgegangen.

Bekannt sind Handstücke für multifunktionale endoskopische Operationsgeräte, die ein Gehäuse, Anschlußmittel, die am Gehäuse angeordnet sind, und die mit Anschlußleitungen, wie elektrischen Leitungen, Flüssigkeitszu- und abflußleitungen, etc. verbindbar sind, wenigstens einen Ultraschall-Wandler, der in dem Gehäuse angeordnet ist, eine Ultraschall-Sonotrode, die die Ultraschallenergie zum distalen Ende leitet, und mindestens eine Schalteinrichtung zur Steuerung von Funktionen des Handstücks aufweisen.

Zum Aufbau und der Funktionsweise einer Sonotrode, soweit er im Rahmen der vorliegenden Anmeldung als bekannt vorausgesetzt wird, wird insbesondere auf die WO-A-91/07917 verwiesen.

Im übrigen wird auf die vorstehend sowie die nachfolgend genannten Druckschriften hinsichtlich der Erläuterung aller hier nicht näher beschriebenen Begriffe ausdrücklich verwiesen.

Die US-A-3 433 226 beschreibt ein Endoskop-Katheter mit einem Ultraschall-Wandler und einer damit verbundenen Sonotrode zur Übermittlung von Schwingungsenergie zu Zwecken medizinischer Behandlungen. Der Wandler ist mit Hochfrequenz betreibbar und die Sonotrode besteht aus metallurgisch miteinander verbundenen akustischen Koppelgliedern, die einen flüssigkeitsleitenden Hohlkanal bilden, wobei das Endglied vorzugsweise flexibel ist und im Betrieb an dessen Ende ein Schwingungsbauch gebildet wird. Ein Gehäuse kann als Griff ausgebildet sein und umgibt vorzugsweise den Wandler, wobei Steckanschlüsse zu Spulen des Wandlers an Gehäuse vorgesehen sind. Schalter werden nicht beschrieben.

Das in der US-A-5 391 144 beschriebene Endoskop ist ein multifunktionales Operationsgerät, das eine Ultraschallfunktion und eine Hochfrequenzfunktion aufweist. Ein Handstück enthält einen Ultraschallgenerator und ist mit auswechselbaren, aus einer Titanlegierung gefertigten Sonotroden verbindbar, wobei eine langgestreckte Sonotrode aus mehreren zusammengeschweißten rohrförmigen Stücken zusammengesetzt sein kann, die einen durchgehenden Kanal bilden, wobei die Verbindungsstellen und das Ende der Sonotrode an den Schwingungsbäuchen der Ultraschallschwingung liegen. Eine Hochfrequenzleitung erstreckt sich durch das Gehäuse und ist mit der Sonotrode verbunden. Schaltkreise des Ultraschallgenerators werden mit Schaltern gesteuert, die am Gehäuse vorgesehen sind, von denen auch einer mit dem Fuss betätigbar sein kann. Ein in eine Ausnehmung der Gehäusewand eingesetzter Schalter umfasst einen Druckknopf, an dessen Unterseite ein erstes Kontaktelement befestigt ist, das bei einer Betätigung des Druckknopfes mit einem am Boden der Ausnehmung befestigten zweiten Kontaktelement in Berührung bringbar ist.

Aus der US-PS 5,391,144 sind somit Instrumente mit folgenden Eigenschaften bekannt:
- endoskopische Anwendung,
- Ultraschallfunktion,
- Hochfrequenzkoagulation,
- Saugfunktion,
- Spülung,
- mehrere Anschlüsse im hinteren Bereich des Handstücks,
- Schalter am Handstück, und
- Dampfsterilisierbarkeit.

Eine ähnliche Vorrichtung ist in der US-PS 5,312,329 beschrieben. Bei diesem ultraschall- und elektrochirurgischen Handstück sind Schalter auf das Handstück aufgesetzt und mit einer separaten Zuleitung versehen.

Die aus der US-PS 5,391,144 und der US-PS 5,312,329 bekannten Vorrichtungen haben jedoch eine Reihe von Nachteilen:
Beispielsweise sind Schalter auf ein bestehendes Handstück aufgesetzt oder mit einer separaten Zuleitung außen am Gehäuse angeklemmt. Die verwendeten Schalter haben die folgenden Nachteile:
   - Der Operateur wird durch den aufgesetzten Schalter und die separate Zuleitung gestört. Die separate Zuleitung wird darüberhinaus häufig beim Einsatz beschädigt.
   - Bei integrierten Schaltern ist häufig eine Kabeldurchführung vorhanden, die mit konstruktivem Aufwand verbunden ist und beim Autoklavieren ein Leckrisiko darstellt. Aus diesem Grund wäre es wünschenswert, Durchführungen möglichst zu vermeiden.
   - Bei eingeschalteter Hochfrequenz (üblicherweise 5000 Vss und 500 kHz) besteht die Gefahr eines Durchschlages. Sind in einem Kabel gemeinsam Hochfrequenzadern und weitere Adern vorhanden, so sind die weiteren Adern durch die kapazitive Kopplung auch auf Hochfrequenzpotential. Aus diesem Grund müssen Schalter zuverlässig gegen Hochfrequenz nach außen isoliert werden. Mit konstruktivem Aufbau ist dies zwar machbar, dabei müßten allerdings relativ große und auftragende Schalter verwendet werden. Kleine, wenig auftragende Schalter wie beispielsweise Folientaster, die auf dem Mantel angebracht sind, sind beim Stand der Technik nicht einsetzbar.
   - Für jede Funktion, die mit dem Operationsgerät ausgeführt werden soll, ist jeweils ein Kabel zur Übertragung und Steuerung erforderlich.

Zudem haben die aus der US-PS 5,312,329 und der US-PS 5,391,144 bekannten Vorrichtungen nur eine unzureichende, wenn nicht sogar überhaupt keine, Isolation zwischen der Hochfrequenz- und der Ultraschallversorgung im Handstück, so daß es unweigerlich zum Übersprechen der Hochfrequenz auf die Ultraschallversorgung im Handstück kommt.

Damit kann es sogar zu einer Leitung der Hochfrequenz zum Ultraschallgenerator kommen, wodurch dieser zerstört werden kann.

Ein weiterer Nachteil der in den genannten Druckschriften beschriebenen Vorrichtungen ist, daß nicht auf eine schwingungsgerechte Konstruktion des Gehäuses und/oder der Sonotrode Rücksicht genommen worden ist.

### Darstellung der Erfindung

Es ist Aufgabe der Erfindung ein Handstück gemäß dem Oberbegriff des Patentanspruchs 1 anzugeben, bei dem zur Erhöhung der Sicherheit für den Anwender sowie die behandelte Person die Schalteinrichtung zuverlässig gegen Hochfrequenz nach außen isoliert ist.

Es ist ferner Aufgabe der Erfindung, ein Handstück für ein Operationsgerät anzugeben, das trotz gleichzeitiger Integration von Ultraschallfunktion und Hochfrequenzanwendung in einem Handstück ausreichend lange funktionstüchtig ist.

Die erfindungsgemäße Lösung dieser Aufgaben ist im kennzeichnenden Teil des Anspruchs 1 angegeben. Weiterbildungen der Erfindung sind Gegenstand der abhängigen Ansprüche.

Die Standzeit der bekannten Sonotroden ist insbesondere dann, wenn die Sondenteile lang sind, vergleichsweise kurz, da sie in der Regel aus Stahldraht gefertigt sind.

Eine Abhilfe könnte zwar die Verwendung von Titan oder Titanlegierungen als Sondenmaterial schaffen. Die Herstellung von Kanälen in derartigen Materialien ist jedoch nur durch Bohren möglich. Tiefbohrungen sind bei akzeptablen Ausschuß nur bis ca. 200 bis 300 mm möglich.

Um dennoch über lange Sondenteile zu verfügen, wird erfindungsgemäß eine Sonotrode eingesetzt, die einen langen und dünnen Sondenteil mit einem Kanal aufweist, der aus mehreren Stücken besteht, die miteinander verschweißt sind. Die Schweißstellen können sich dabei insbesondere in den Schwingungsbäuchen der Ultraschallwelle befinden. Damit ist es möglich, Sonden aus Titan oder Titanlegierungen, wie beispielsweise Ti6A14V herzustellen, bei denen der Durchmesser des Kanals ca. 2 mm, die Dicke des Sondenteils weniger als 4 mm und die Länge mehr als 30 cm betragen. Die gebohrten Rohrstücke sind dabei bevorzugt so angedreht, daß sie selbstzentrierend verschweißt werden können.

Insbesondere kann die Sonotrode in an sich bekannter Weise eine Verdickung aufweisen, die mit dem Sondenteil verschweißt und mit dem Gehäuse verschraubt ist. Bezüglich der genauen Ausgestaltung wird auf die eingangs genannte WO-A-91/07917 verwiesen.

Weiterhin ist es von Vorteil, wenn ein einziges Anschlußmittel vorgesehen ist, und sämtliche Handstück-Anschlüsse der Anschlußleitungen an dem einen Anschlußmittel angebracht sind. Ein Handstück mit diesen Merkmalen erspart zusätzliche außerhalb des Handstücks liegende Kabel. Sämtliche Anschlüsse werden an das eine Anschlußmittel, das an einem Ende des Handstücks bzw. des Gehäuses angeordnet ist, angeschlossen.

Vorteilhafterweise sind sämtliche Anschlußleitungen, die nicht als Einweg-Leitungen -Einweg-Leitungen sind typischerweise Fluid-Leitungen - ausgeführt sind, in einem Kabelmantel eingebracht. Dieses ermöglicht eine besonders einfache Handhabung des Handstücks.

Als Ultraschall-Wandler können bevorzugt Piezowandler verwendet werden. Da die Schwungmassen bei den Piezowandlern beim Stand der Technik in der Regel an die elektrische Masse gelegt werden, ist es üblich, eine gerade Anzahl von Piezoscheiben und damit eine ungerade Anzahl von Elektrodenflächen zu benutzen.

Um den Patientenableitstrom zu minimieren, sollten dagegen vorteilhafterweise alle Piezoscheiben von der Schwungmasse isoliert sein. Bei einer geraden Anzahl von Elektrodenflächen, d.h. bei gleich vielen positiven wie negativen Flächen kompensieren sich im wesentlichen die positiven und negativen Ströme, die kapazitiv zur Schwungmasse koppeln und deshalb durch Berührung zum Patienten gelangen können.

Weiterhin ist es von Vorteil, wenn der Ultraschallgenerator einen floatenden, symmetrischen Ausgang aufweist, so daß aufgrund der geraden Zahl von Elektrodenflächen sich aus Symmetriegründen die kapazitiv an den Patienten gekoppelten Ströme zu einem minimalen Ableitstrom kompensieren.

Die Schalteinrichtung weist wenigstens einen Schaltsensor und mindestens ein Betätigungselement auf, wobei das Betätigungselement kein Teil aufweist, das mit dem Schaltsensor in direkten mechanischen Kontakt bringbar ist. Insbesondere wird dadurch auch keine elektrische Verbindung hergestellt, so daß eine Hochfrequenzkopplung schon im wesentlichen zwischen Schalter und Betätigungselement vermieden wird.

Erfindungsgemäß ist nämlich festgestellt worden, daß die beim Stand der Technik aufgetretenen Nachteile bereits dadurch vermieden werden können, daß eine Schaltfunktion durch Fernwirkung durch die Gehäusewand, wie beispielsweise magnetisch, induktiv, elektromagnetisch, optisch und/oder akustisch übermittelt wird.

So ist es möglich, daß in dem beweglichen Betätigungselement, außerhalb des Gehäuses oder in die Gehäusewand integriert, ein Magnet angeordnet ist, der im Inneren des hermetisch dichten Gehäuses beispielsweise einen Reed-Kontakt oder eine Hall-Sonde für die Ultraschallfunktion schaltet.

Dabei kann das Handstück derart ausgebildet sein, daß das Betätigungselement einen Magneten aufweist, dessen Magnetfeld sich durch Betätigung des Betätigungselementes am Ort des Schalters ändert, und daß der Schalter magnetfeldabhängig schließt bzw. öffnet.

Weiterhin ist es von Vorteil, wenn die Schalteinrichtung ein Betätigungselement aufweist, mit dem nicht nur Schaltfunktionen ausgeführt werden können, sondern mit dem auch Eigenschaften eines Schaltkreises variiert werden können. Durch diese Maßnahme können mehrere Funktionen durch das Betätigungselement variiert werden. Diese Funktionen sind beispielsweise die Amplitude, Einschalten oder Ausschalten der Spülung, Variation des Saugdrucks, etc.

Hierzu kann der Schaltkreis Schwingkreise mit unterschiedlichen Resonanzfrequenzen aufweisen; durch Betätigen des Betätigungselementes wird jeweils mindestens ein Schwingkreis aktiviert. Bei dieser Ausführung wird außerhalb des Gehäuses oder in die Gehäusewandung integriert ein Schaltkreis angeordnet, der die Befehle induktiv ins Gehäuseinnere überträgt. Außerhalb oder in der Gehäusewandung und innerhalb des Gehäuses sind beispielsweise Induktionsspulen angeordnet. Hierdurch wird die äußere Spule Teil eines Schwingkreises, indem sie über Tastenkontakte mit verschiedenen Kondensatoren parallel geschaltet wird. Dabei entsteht für jede gewünschte Funktion eine charakteristische Resonanzfrequenz. Die innere Spule dient als Sensor für die Resonanzfrequenz des Schwingkreises. Eine Schaltung erkennt diese Frequenz und leitet den entsprechenden Befehl weiter.

Bei der oben genannten Ausgestaltung ist es ausreichend, wenn nur zwei Signalleitungen im Zuleitungskabel des Handstücks vorhanden sind. Die beiden Leitungen können beispielsweise für die Einschaltfunktion sowie für die Temperaturüberwachung im Handstück und die Handstück-Typ-Kodierung genutzt werden. Hierzu wird beispielsweise mit Dioden ein Netzwerk aufgebaut, das in der einen Stromrichtung den Reed-Schalter und in der Gegenrichtung die Temperatur über einen Thermostatschalter abfragt. Die Kodierung wird durch zusätzliche Elemente, wie einen in Serie zum Thermostaten geschalteten Widerstand festgelegt.

Um im Falle des normalen Betriebes oder im Falle eines Defektes des Handgerätes eine ausreichende Sicherheit für den Operateur und den Patienten zu gewährleisten, ist eine ausreichende Isolation notwendig:

Normiert ist für die Trennung von 5000 Vss eine Luftstrecke von 16 mm und eine Kriechstrecke von 23 mm.

Erfindungsgemäß wird deshalb die Isolation zwischen Hochfrequenz- und Ultraschallversorgung in das Handstück verlegt oder der Ultraschallgenerator isoliert ausgeführt.

Insbesondere können zur Isolation zwischen Hochfrequenz- und Ultraschallversorgung im Handstück etwaige Kriechstrecken mit Polyimidband und/oder Keramik und/oder Silikon und/oder GFK und/oder CFK verlängert werden. Dabei wird die Ultraschallfunktion nicht wesentlich beeinflußt. Durch die Verwendung von Silikon werden vorteilhafterweise gleichzeitig Querschwingungen gedämpft und die Verdrehsicherung verbessert.

Bei einer weiteren Ausgestaltung erfolgt die Isolation zumindest teilweise mit einem Schrumpfschlauch (Polyvinylidenfluorid (PVDF), Silikon etc.) und/oder einem Isolationsring.

Bei einer weiteren bevorzugten Ausgestaltung besteht das Gehäuse des Handstücks aus Kunststoff. Kunststoff hat u.a. den Vorteil, bei geringem Gewicht gut zu isolieren.

Als Kunststoff kommt beispielsweise PEEK in Frage. Dieser Kunststoff ist uneingeschränkt sterilisierbar. Eine weitere Möglichkeit ist ein Verbundkunststoffgehäuse, das beispielsweise durch eine Faserwickeltechnik, wie beispielsweise glas-, kohle-, oder kevlarverstärkter Kunststoff, hergestellt wird. Die Verbundkunststoffgehäuse sind bevorzugt dünnwandig und haben somit einen kleinen Außendurchmesser und ein noch geringeres Gewicht.

Vorteilhafterweise ist - beispielsweise an dem Handstück oder zwischen Handstück und einer Pumpeneinheit - ein Umschalter angebracht, dessen Betätigung bewirkt, daß die Funktion der Absaug- und Spülleitungen vertauscht wird. Während einer Operation kann es nämlich von Vorteil sein, wenn der Spülkanal zum Absaugen verwendet wird, zum Beispiel wenn der Absaugkanal als Instrumentenkanal verwendet wird. Auch bei verstopfter Saugleitung kann kurzfristig während der Operation ein Vertauschen der Spül- und Absaugleitung Abhilfe schaffen. Wird ferner durch die Absaugleitung gespült, so entsteht ein gerichteter Strahl, der bei gleichzeitigem Ultraschall eine vielfache Reichweite besitzt (und zwar etwa 30 cm) und teilweise fein vernebelt wird.

Um eine zuverlässige Funktion zu gewährleisten, werden weiterhin erfindungsgemäß Befestigungen, Halterungen bzw. Ankopplungen von Schläuchen, Rohren, elektrischen Anschlüssen und/oder Dichtungen in der Nähe von Schwingungsknoten (Abstand weniger als λ/8) oder in Schwingungsknoten angebracht. Dadurch wird die Verlustleistung, die Ermüdung und die Erwärmung der Teile minimiert. Als Beispiele für eine derartige Konstruktion seien das Saugrohr im Horn, das Spülrohr im Bolzen, die Keramikaufhängung im Mantel, die Abstützungen am Horn und an der verlängerten Sonde, die Anlötstelle der Piezoelemente nahe des Knotens sowie der Hochfrequenzanschluß nahe des Knotens genannt.

Üblicherweise wird das Horn einer Sonotrode an der Spitze getrimmt, bis es auf der richtigen Frequenz schwingt. Bei dieser Vorgehensweise ist das Horn allerdings nicht in der Länge definiert und damit nicht immer mit einem das Horn umgebenden Mantel definierter Länge kombinierbar. Bei einer genau definierten Länge des Horns ist es nicht notwendig, daß der Mantel dem Horn angepaßt wird. Die Resonanzfrequenz wird beispielsweise erhöht, wenn der Außendurchmesser an der Ankoppelstelle, also an einem Schwingungsbauch abgedreht wird, was eine Reduktion der Schwungmasse bewirkt. Die Frequenz wird tiefer, wenn bei einem Knoten abgedreht wird, da die Federkonstante so reduziert wird.

Um für bestimmte Operationstechniken (z.B. Prostataresektion) einen effizienten Gewebeabtrag bei einer Kombination von Ultraschall- und Hochfrequenzenenergie-Eintrag zu erzielen, ist es weiterhin von Vorteil, wenn im Bereich des distalen (patientennahen) Endes der Sonotrode ein abtragungsverstärkendes Element angeordnet ist. Dieses kann insbesondere ein Ringmantel sein, der als schneidender Schlingring ausgebildet ist. Dabei kann der Ringmantel auch an einer Sonde angebracht sein, die keine Bohrung aufweist.

Aufgrund der großen Beschleunigung an der Spitze ist ein Design mit einer hohen Steifigkeit wünschenswert. Vorteilsweise folgt dabei die Abwicklung des Ringmantels bzw. des Schlingrings einer exponentiellen oder quadratischen Kurve. Insbesondere Drahtschlingen mit konstantem Querschnitt sind wegen ihrer Trägheit und Nachgiebigkeit nicht genügend wirksam für die Ultraschallübertragung.

Wenn die Mittelsenkrechte des Ringmantels und die Symmetrieachse der Sonotrode parallel zueinander ausgerichtet sind, wird eine hohe Steifigkeit erreicht und somit ein hoher Abtrag erzielt.

Vorteilsweise ist der Ringmantel mit der Sonotrode verschweißt.

Um die Standzeit der Sonde bzw. deren Haltbarkeit zu erhöhen wird vorteilhafterweise die Spitze der Sonotrode und/oder der Ringmantel oberflächenbehandelt oder aus Hartmetall gefertigt. Eine derartige Spitze eignet sich besonders für die Entfernung von harten Steinen. Es kann ferner ein Hartmetall-Einsatz angelötet oder angeschweißt werden.

Vorteilsweise wird weiterhin eine Absaugleitung verwendet, die gerade ist. Der gerade Verlauf erlaubt die Verwendung der Absaugleitung als Instrumentenkanal und/oder die Einführung einer Beobachtungsoptik und/oder eines Lichtleiters für Laserbehandlungen.

Bei einem multifunktionalen Operationsgerät gemäß dem Stand der Technik kann es vorkommen, daß durch Kapazitäten zwischen den Leitern die Hochfrequenz-Hochspannung, die an die Sonde angelegt wird, in den Ultraschallgenerator geleitet wird, so daß dieser zerstört wird.

Dies kann umso eher geschehen, je näher die Hochfrequenzleitungen bei den Ultraschalleitungen liegen. Ein Überspringen bzw. Koppeln der Hochfrequenz ist bei einem multifunktionalen Operationsgerät, das nur ein Handstück für sämtliche verwendeten Funktionen aufweist, beim Stand der Technik nicht zu verhindern.

Erfindungsgemäß ist erkannt worden, daß sich die hochfrequenten Signalanteile mit über 100 kHz (hier sinnvolle Bereiche: Ultraschall: 18-100 kHz, HF: 100 kHz - 5 MHz) auskoppeln lassen.

Bevorzugt wird die Erfindung bei einem multifunktionalen endoskopischen Operationsgerät mit einer Ultraschallfunktion zum Zertrümmern von Gewebe, einer Hochfrequenzfunktion zum zusätzlichen Schneiden und/oder Koagulieren, und einer Spül- und/oder Absaugfunktion realisiert. Die Funktionen werden durch den Anwender mit einem erfindungsgemäßen Handstück gesteuert. es wird Gewebe verflüssigt oder zerkleinert oder im Bereich der Mündung eingesaugt und abgerissen.

Unter Zertrümmern von Gewebe wird verstanden, daß Gewebe verflüssigt oder verkleinert und im Bereich eher Mündung eingesaugt und abgerissen wird. Es wird Volumen bzw. Material entfernt. Dadurch kann man das erfindungsgemäße Handstück auch indirekt zum Schneiden benutzen mit einem Schnittspalt von 4 mm. Der Ringmantel schneidet ohne Volumenabtrag. Er wird zum Schaben/Hobeln eingesetzt).

Weiterhin ist es bevorzugt, wenn die Abkoppelung durch mindestens ein induktives Element und insbesondere durch eine Kompensationsspule erfolgt. Dies hat den Vorteil, daß beispielsweise die verwendeten Leitungen gemeinsam um den Kern der Kompensationsspule gewickelt werden können und sich so deren Magnetfelder im Kern kompensieren. Die Spule unterdrückt dabei die "common-mode"-Hochfrequenz und ist für die differentiellen Ultraschallströme ohne Wirkung.

Vorteilhafterweise werden die spannungsführenden Kabel zwischen Ultraschall-Generator und Kompensationsspule durch Kapazitäten gegen Erde entladen. Der HF-Spannungsabfall erfolgt hierbei in der Kompensationsspule.

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird.
Es zeigen:
- Fig. 1:: Teil eines Handstücks in schematischer Darstellung mit Betätigungselement und Piezoelement,
- Fig. 2:: Querschnitt durch einen Teil eines Handstücks,
- Fig. 3:: Querschnitt durch einen anderen Teils eines Handstücks,
- Fig. 4:: Wie Fig. 3 nur in anderer Ausführungsform,
- Fig. 5:: Wie Figuren 3 und 4 in anderer Ausführungsform,
- Fig. 6:: Übersichtsskizze eines erfindungsgemäßen Operationsgerätes mit Kompensationsspule,
- Fig. 7:: Sonotrode mit einer Länge von 4*λ/2,
- Fig. 8:: Sondenspitze mit Ringmantel (links in zylindrischer Ausgestaltung und rechts in exponentieller Ausgestaltung),
- Fig. 9:: Sondenspitze mit Ringmantel in zylindrischerAusgestaltung in perspektivischer Darstellung.

### Darstellung von Ausführungsbeispielen

In den folgenden Figuren sind jeweils gleiche oder entsprechende Teile mit den selben Bezugszeichen bezeichnet, so daß auf eine erneute Vorstellung verzichtet wird, und lediglich die Abweichungen der in diesen Figuren dargestellten Ausführungsbeispiele gegenüber dem vorigen Ausführungsbeispielen erläutert werden:

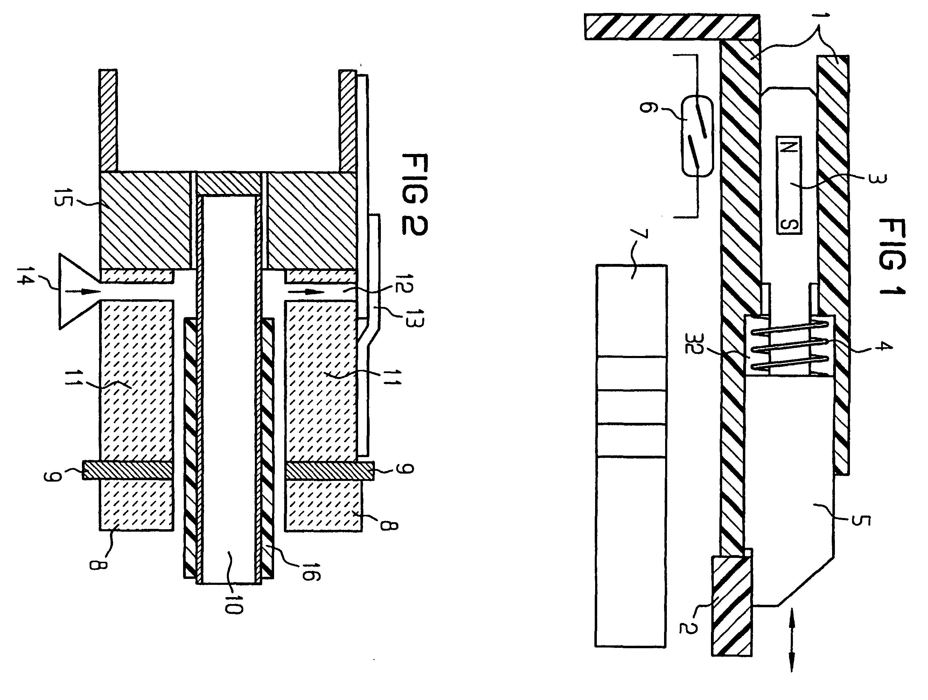

In Fig. 1 wird ein Teil eines erfindungsgemäßen Handstücks im Querschnitt in schematischer Darstellung gezeigt. Durch diese Figur soll insbesondere das Schalten durch Fernwirkung erläutert werden.

Mit 1 ist das Gehäuse-Hinterteil und mit 2 das Gehäuse-Vorderteil benannt. Mit einem Permanentmagnet 3, der mit einem Schieber 5, in diesem Fall das Betätigungselement, verbunden ist, wird ein Reed-Relais 6 bzw. ein Reed-Kontakt 6 durch Verschieben des Schiebers 5 und damit des Magneten 3 geschaltet. Durch eine Feder 4, die wie auch der Schieber 5 in eine abgesetzte Bohrung 32 eingelassen ist, wird der Schieber wieder in seine Ausgangsposition gedrückt. Der Schieber 5 ist vorne ausgefräst, so daß er durch das Gehäuse-Vorderteil 2 gegen Herausfallen gesichert ist. Der Doppelpfeil deutet die Bewegung des Schiebers 5 an. Nur bei Betätigung des Schiebers 5 wird also beispielweise die Ultraschallanregespannung auf das Ende des Handstücks gelegt. Bei Zerlegung des Gehäuses läßt sich der Schieber zur Reinigung herausnehmen. In Fig. 1 sind ferner Piezoelemente 7 angedeutet.

Die Figuren 2 bis 5 deuten verschiedene Möglichkeiten der Isolation zwischen Ultraschall und Hochfrequenz an. Im Betrieb ist es insbesondere bei einem Defekt aber auch im Normalbetrieb notwendig, die Hochfrequenz von dem Ultraschall elektrisch zu isolieren. Erfindungsgemäß wird in diesem Ausführungsbeispiel eine Kompensationsspule verwendet. Aus diesem Grund und da das verwendete Kabel, in dem alle Leitungen untergebracht sind, die Hochfrequenz und den Ultraschall kapazitiv koppelt, ist die Spannung über der Isolation im Handstück im Normalfall nur ein Bruchteil der vollen Hochfrequenzspannung. Zwischen dem Stromnetz und dem Patienten muß eine Isolation bestehen, deren Größe von der höchsten angelegten Spannung abgeleitet wird. Normiert sind für die Trennung von 5000 Vss eine Luftstrecke von 16 mm und eine Kriechstrecke von 23 mm. Dieses ist im Generator nur schwer zu realisieren, weswegen die Isolation in das Handstück verlegt wird. Sofern eine Isolation im Ultraschallgenerator realisiert wird, fungiert die Isolation im Handstück als zusätzliche Sicherheit.

Durch spezielle Konstruktionen werden die Kriechstrekken zwischen Hochfrequenz und Ultraschall verlängert, ohne daß dabei die Ultraschallfunktion wesentlich beeinflußt wird. Am hinteren Konverter- bzw. Wandlerende, das in Fig. 2 durch den Piezo 8 und die Elektrode 9 angedeutet ist, ist eine Spannmutter 15 mit einer Verlängerungslippe versehen, die mit einem Isolierband 13 umwickelt ist. Die Spannmutter 15 mit ihrem Bolzen 10 bzw. dessen zylindrischer Rand liegen dabei auf Hochfrequenz. Eine Isolation findet ferner über eine Keramik 11 und eine Silikonmasse 14 statt. Dazu wird ein Silikonverguß 14 in Pfeilrichtung durch eine Befüllöffnung 12a so lange eingepreßt, bis sie an der Entlüftung 12b austritt.

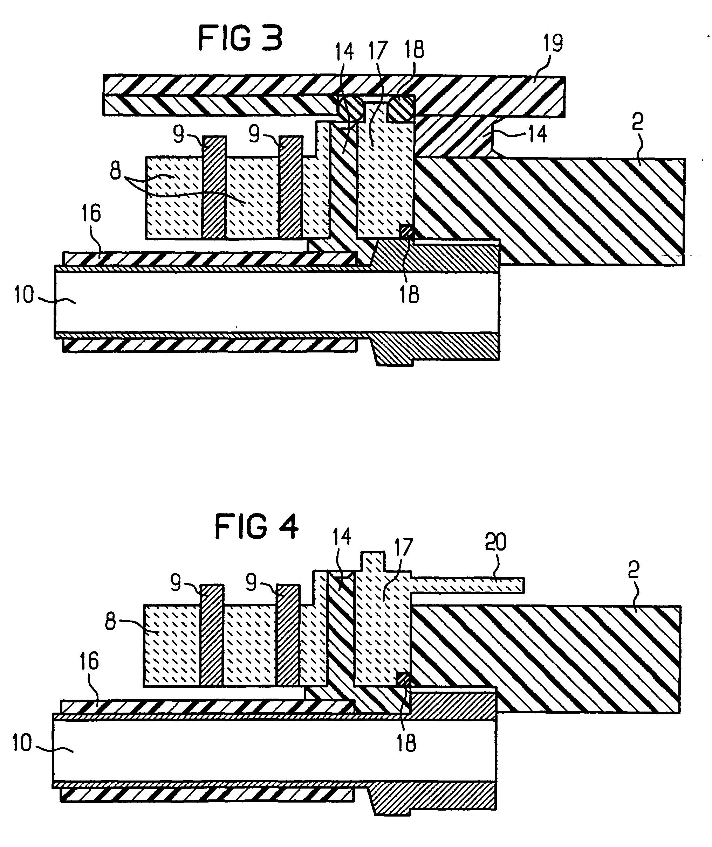

Die Aufhängung ist beispielsweise aus isolierender Keramik 17 gefertigt (siehe Fig. 3). Der Raum zwischen Vorderteil 2 und Gehäusewand 19 wird zur Unterbrechung der Kriechstrecke mit Silikon 14 vergossen. Durch diese Maßnahme werden gleichzeitig Querschwingungen gedämpft und eine Verdrehsicherung gewährleistet. Der Bolzen 10 und die Piezos 8 sind durch einen Silikonschlauch 16 isoliert. Die Enden des Schlauches 16 sind mit den jeweiligen Keramikteilen 11 bzw. 17 vergossen. Der Verguß geschieht durch entsprechende Einspritz- und Entlüftungsöffnungen 12a und 12b. Die Silikon-Vergußmasse 14 geht mit dem Schlauch 16 eine perfekte vulkanisationsartige Verbindung ein. Die Gehäusewand 19 ist beispielsweise aus Kunststoff.

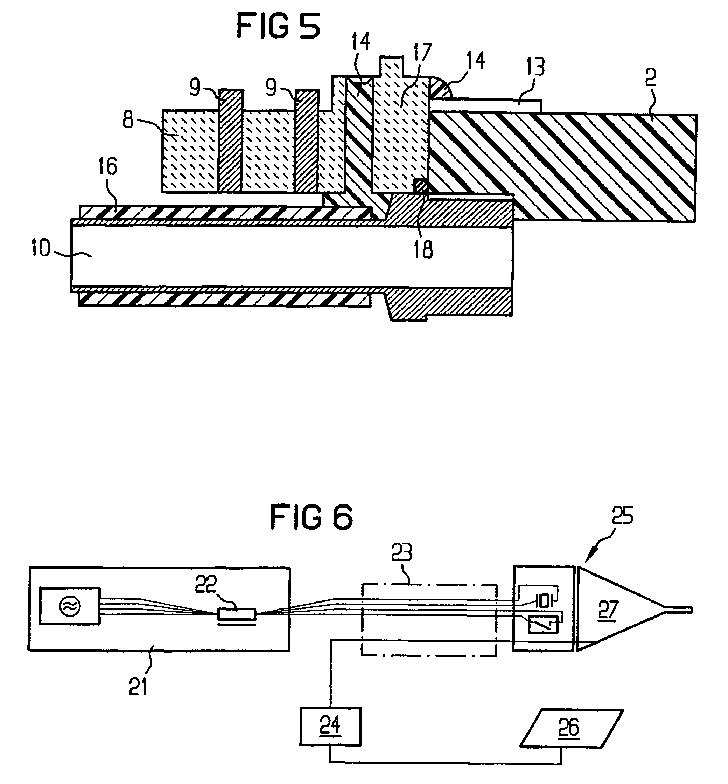

Eine weitere Isolationsmöglichkeit an der Aufhängung ist in Fig. 4 gezeigt. Dort wird die Kriechstrecke mit einem Isolationsring 20 verlängert. In Fig. 5 ist eine weitere Möglichkeit der Isolation gezeigt. Hier ist das Vorderteil 2 mit Isolierband 13 umwickelt. Die Anstoßstelle zur Keramikaufhängung 17 wird dann mit Silikonmasse 14 versiegelt.

In Fig. 6 ist schematisch ein multifunktionales endoskopisches Operationsgerät gezeigt. Der Ultraschallspannungsgenerator 21 weist eine Kompensationsspule 22 auf. Die Leitungen von dem Ultraschallspannungsgenerator 21 als auch die Leitung vom Hochfrequenzgenerator 24 verlaufen durch ein gemeinsames Kabel 23 zum Handstück 25. Im Handstück 25 sind schematisch der Piezowandler, das Reed-Relais und die Sonotrode 27 dargestellt. Ferner ist eine Neutralelektrode 26 in Fig. 6 gezeigt.

Die elektrischen Anschlüsse (Schaltleitungen, Hochfrequenz, Ultraschall), die in einem gemeinsamen Kabel 23 integriert sind, sind am proximalen Ende aufgespleißt und zwar in einen Teil mit Ultraschall- und Schaltleitungen für den Ultraschallspannungsgenerator und einen Teil mit der Hochfrequenzleitung für den Hochfrequenzgenerator.

Um den Kern der Kompensationsspule 22 sind die vier Leitungen (hier 2 Ultraschall- und 2 Signalleitungen) gewickelt, so daß die Spule die "Common-Mode"-Hochfrequenz unterdrückt und die differentiellen Ultraschall- und Signalströme nicht beeinflußt.

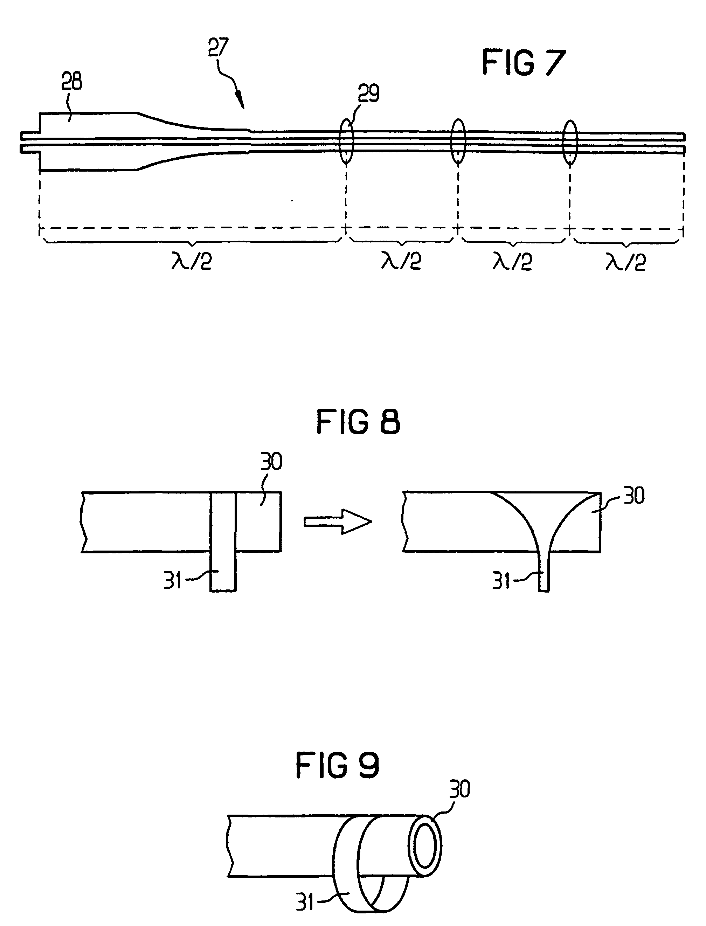

Das erfindungsgemäße Handstück eignet sich insbesondere für den Einsatz mit Trokaren, bei denen Sonotroden Verwendung finden, die in die Länge gestreckt sind. Dieses wird besonders durch Fig. 7 deutlich, in der eine Sonotrode 27 dargestellt ist. In Fig. 7 ist mit 28 ein Schraubengewinde angedeutet, mit dem die Sonotrode an dem Gehäuse des Handstücks befestigt ist, so daß insbesondere die Kopplung zu den Piezoelementen stattfindet. Die dargestellte Sonotrode hat eine Länge von vier halben Wellenlängen. In den Schwingungsbäuchen sind Schweißstellen angebracht. Die Sonotrode aus Fig. 7 hat am patientennahen Ende einen Schwingungsbauch.

Die Schweißstellen sind notwendig, da die verwendeten Titanlegierungen nicht als passendes Rohr erhältlich sind, und die Sonden aus diesem Grund tiefgebohrt werden müssen. Verlängerte Sonden, wie diejenigen, die in der Endoskopie Anwendung finden und beispielsweise in Trokare eingesetzt werden sollen, sind aus verschiedenen Stücken zusammengesetzt, da einseitige Tiefbohrungen bei vernünftiger Fehlerquote (20%) nur bis zu 200 mm möglich sind. Erfindungsgemäß ist festgestellt worden, daß die gewünschten Ultraschalleigenschaften und die Haltbarkeit bei wenig bis gar keiner Nachbearbeitung durch Schweißen erreicht werden kann. Zudem ist die Schweißtechnik kostengünstig und zuverlässig, d.h. es wird wenig Ausschuß produziert.

Um bei gewissen Operationstechniken (Prostataresektion) einen effizienten Gewebeabtrag zu erzielen, wird erfindungsgemäß ein Ringmantel an die Sondenspitze angebracht. Fig. 8 zeigt dazu in seitlicher Darstellung eine Sondenspitze 30 mit einem Ringmantel 31. Links ist eine zylindrische Ausführungsform dargestellt, die in Fig. 9 noch einmal perspektivisch aufgeführt ist. Auf der rechten Seite der Fig. 8 ist ein Ringmantel 31 dargestellt, der eine erhöhte Steifigkeit durch eine exponentiell verlaufende Form aufweist. Eine erhöhte Steifigkeit ist aufgrund der sehr hohen Beschleunigung und damit hohen Belastung des Materials in der Ultraschallanwendung notwendig.

### Bezugszeichenliste

- 1: Gehäuse-Hinterteil
- 2: Gehäuse-Vorderteil
- 3: Magnet
- 4: Feder
- 5: Schieber
- 6: Reed-Kontakt
- 7: Piezoelemente
- 8: Piezo
- 9: Elektrode
- 10: Bolzen
- 11: Keramik
- 12: Entlüftung
- 13: Kapton
- 14: Silikonmasse
- 15: Spannmutter
- 16: Silikonschlauch
- 17: Keramikaufhängung
- 18: O-Ring
- 19: Kunststoff-Gehäusewand
- 20: Isolationsring
- 21: Ultraschallspannungsgenerator
- 22: Kompensationsspule
- 23: Kabel
- 24: Hochfrequenzgenerator
- 25: Handstück
- 26: Patientenelektrode
- 27: Sonotrode
- 28: Schraubgewinde
- 29: Schwingungsknoten
- 30: Sondenspitze
- 31: Ringmantel
- 32: abgesetzte Bohrung

## Patentansprüche

1. Handstück (25) für ein multifunktionales endoskopisches Operationsgerät, das mindestens eine Ultraschallfunktion aufweist, mit
- einem Gehäuse,
- Anschlussmitteln, die am Gehäuse angeordnet und mit elektrischen Leitungen sowie Flüssigkeitsleitungen verbindbar sind,
- wenigstens einem in dem Gehäuse angeordneten Ultraschall-Wandler (7),
- einer Ultraschall-Sonotrode (27), welche Ultraschallenergie zu einem distalen Ende des Operationsgeräts leitet, und
- mindestens einer ohne separate Zuleitung und Kabeldurchführungen am Handstück vorgesehenen Schalteinrichtung mit einem Betätigungselement und einem zugeordneten Schaltkreis zur Steuerung von Funktionen des Handstücks,
**dadurch gekennzeichnet,**
**dass** die Schalteinrichtung wenigstens einen Schaltsensor aufweist, und dass das Betätigungselement kein Teil aufweist, das mit dem jeweiligen Schaltsensor in direktem mechanischen Kontakt bringbar ist.

2. Handstück nach Anspruch 1,
**dadurch gekennzeichnet, dass** die Sonotrode (27) einen langgestreckten Sondenteil mit einem Kanal aufweist, der aus mehreren Stücken besteht, die miteinander verschweißt sind, und dass die Schweißstellen sich in den Schwingungsbäuchen der Ultraschallwelle befinden.

3. Handstück nach Anspruch 1 oder 2,
**dadurch gekennzeichnet, dass** zumindest der Sondenteil aus Titan oder einer Titanverbindung besteht, und dass der Durchmesser des Kanals ca. 2 mm, die Dicke des Sondenteils weniger als 4 mm und die Länge mehr als 30 cm betragen.

4. Handstück nach einem der Ansprüche 1 bis 3,
**dadurch gekennzeichnet, daß** die Sonotrode proximalseitig eine Verdickung aufweist, die mit dem Sondenteil verschweißt und mit dem Gehäuse verschraubt ist.

5. Handstück nach einem der Ansprüche 1 bis 4,
**dadurch gekennzeichnet, daß** ein einziges Anschlußmittel vorgesehen ist, und
daß sämtliche Handstück-Anschlüsse der Anschlußleitungen an dem einen Anschlußmittel angebracht sind.

6. Handstück nach Anspruch 7,
**dadurch gekennzeichnet, daß** Informationen vom Betätigungselement magnetisch, induktiv, elektromagnetisch, optisch und/oder akustisch zum Schaltsensor übermittelt werden, der mit dem Schaltkreis verbunden ist.

7. Handstück nach Anspruch 6,
**dadurch gekennzeichnet, daß** das Betätigungselement einen Magneten (3) aufweist, dessen Magnetfeld am Ort des Schaltsensors sich durch Betätigung des Betätigungselementes ändert, und daß der Schaltsensor magnetfeldabhängig schließt bzw. öffnet.

8. Handstück nach Anspruch 6 oder 7,
**dadurch gekennzeichnet, daß** der Schaltsensor ein Reed-Relais oder eine Hall-Sonde ist.

9. Handstück nach einem der Ansprüche 1 bis 7,
**dadurch gekennzeichnet, daß** die Schalteinrichtung die Variation von Eigenschaften wenigstens eines Schaltkreises erlaubt.

10. Handstück nach Anspruch 8,
**dadurch gekennzeichnet, daß** das bzw. die Betätigungselemente Schwingkreise mit unterschiedlichen Resonanzfrequenzen aufweisen, und
daß durch Betätigen des Betätigungselementes jeweils mindestens ein Schwingkreis aktiviert wird.

11. Handstück nach Anspruch 9 oder 10,
**dadurch gekennzeichnet, daß** im Handstück eine Spule und eine Schaltung vorgesehen sind, die induktiv die Resonanzfrequenz des aktivierten Schwingkreises erfassen.

12. Handstück nach einem der Ansprüche 1 bis 11,
**dadurch gekennzeichnet, daß** in der Anschlußleitung nur zwei Signalleitungen für die Einschaltfunktion sowie weitere Funktionen, wie die Temperaturüberwachung und die Handstück-Kodierung vorgesehen sind.

13. Handstück nach einem der Ansprüche 1 bis 12,
**dadurch gekennzeichnet, daß** zusätzlich eine HF-Einrichtung vorgesehen ist.

14. Handstück nach Anspruch 13,
**dadurch gekennzeichnet, daß** zur Isolation zwischen Hochfrequenz- und Ultraschallversorgung im Handstück etwaige Kriechstrecken verlängert oder unterbrochen sind.

15. Handstück nach Anspruch 14,
**dadurch gekennzeichnet, daß** die Isolation zumindest teilweise mit einem PVDF-Schlauch und/oder einem Isolationsring erfolgt.

16. Handstück nach Anspruch 14 oder 15,
**dadurch gekennzeichnet, daß** es ein Gehäuse aus Kunststoff aufweist.

17. Handstück nach einem der Ansprüche 2 bis 16,
**dadurch gekennzeichnet, daß** der Kanal in der Sonotrode geradlinig durch das Handstück bis zu einem Fluid-Anschluß verläuft.

18. Handstück nach Anspruch 17,
**dadurch gekennzeichnet, daß** der Kanal zum Saugen oder Spülen dient.

19. Handstück nach Anspruch 18,
**dadurch gekennzeichnet, daß** ein Umschalter vorgesehen ist, durch dessen Betätigung von Absaugen auf Spülen und umgekehrt umgeschaltet wird.

20. Handstück nach einem der Ansprüche 1 bis 19,
**dadurch gekennzeichnet, daß** Befestigungen, Halterungen bzw. Ankopplungen von Schläuchen, Rohren, elektrischen Anschlüssen und/oder Dichtungen näher als λ/8 zu Schwingungsknoten angebracht sind.

21. Handstück nach einem der Ansprüche 1 bis 20,
**dadurch gekennzeichnet, daß** im Bereich des distalen (patientennahen) Endes der Sonotrode (27) ein abtragungsverstärkendes Element angeordnet ist.

22. Handstück nach Anspruch 21,
**dadurch gekennzeichnet, daß** das abtragungsverstärkende Element ein schneidender Schlingring ist.

23. Handstück nach Anspruch 22,
**dadurch gekennzeichnet, daß** die Mittelsenkrechte des schneidenden Schlingrings und die Symmetrieachse der Sonotrode (27) parallel zueinander angeordnet sind.

24. Handstück nach Anspruch 22 oder 23,
**dadurch gekennzeichnet, daß** der schneidende Schlingring in zumindest einer Projektion an der Befestigungsbasis breiter ist als im gegenüberliegenden Bereich.

25. Handstück nach Anspruch 24,
**dadurch gekennzeichnet, daß** die Kontur des Schlingrings einer exponentiellen oder quadratischen Kurve folgt.

26. Handstück nach einem der Ansprüche 22 bis 25,
**dadurch gekennzeichnet, daß** der Schlingring mit der Sonotrode (27) verschweißt ist.

27. Handstück nach einem der Ansprüche 1 bis 26,
**dadurch gekennzeichnet, daß** die Spitze der Sonotrode (27) und/oder die Schneide des Schlingrings aus Hartmetall bestehen und/oder beschichtet sind.

28. Handstück nach einem der Ansprüche 17 bis 27,
**dadurch gekennzeichnet, daß** die Absaugleitung als Instrumenten- und/oder Beobachtungskanal verwendet wird.

29. Handstück nach einem der Ansprüche 1 bis 28,
**dadurch gekennzeichnet, daß** vor einem Ultraschallgenerator (28), der die Spannungsversorgung für die Ultraschallfunktion bereitstellt, hochfrequente Signalanteile ausgekoppelt werden.

30. Handstück nach Anspruch 29,
**dadurch gekennzeichnet, daß** die hochfrequenten Signalanteile durch mindestens ein induktives Element ausgekoppelt werden.

31. Handstück nach Anspruch 30,
**dadurch gekennzeichnet, daß** eine Kompensationsspule (22) als induktives Element dient.

32. Handstück nach Anspruch 31,
**dadurch gekennzeichnet, daß** die spannungsführenden Kabel um den Kern der Kompensationsspule (22) gewickelt sind und sich deren Magnetfelder im Kern kompensieren.

33. Handstück nach einem der Ansprüche 30 bis 32,
**dadurch gekennzeichnet,** die spannungsführenden Kabel durch Kapazitäten gegen Erde gedämpft sind.

34. Handstück nach einem der Ansprüche 1 bis 33,
**dadurch gekennzeichnet, daß** der Ultraschall-Wandler aus Piezoscheiben besteht.

35. Handstück nach Anspruch 34,
**dadurch gekennzeichnet, daß** die Zahl der Piezoscheiben gerade'ist, und
daß die Piezoscheiben von der Schwungmasse isoliert sind.

36. Handstück nach Anspruch 34 oder 35,
**dadurch gekennzeichnet, daß** der Generator (21) der den Ultraschall-Wandler ansteuert, einen floatenden symmetrischen Ausgang aufweist.

## Claims

1. Handpiece (25) for a multifunctional endoscopic surgical instrument having at least one ultrasound facility, comprising:
- a housing;
- connecting means disposed on the housing and adapted to be connected to electrical and liquid supply lines;
- at least one ultrasonic transducer (7) disposed inside the housing;
- an ultrasonic sonotrode (27) for conveying ultrasonic energy to a distal end of the surgical instrument; and
- at least one switch unit provided on the handpiece without separate leads and cable leadthroughs, the switch unit having an actuating element and an associated electrical circuit for controlling operations of the handpiece;
**characterized in that**
the switch unit has at least one switch sensor; and that the actuating element comprises no component that is adapted to be put into direct mechanical contact with the respective switch sensor.

2. Handpiece according to claim 1,
**characterized in that** the sonotrode (27) has an elongate probe portion with a channel, the probe portion consisting of a plurality of pieces that are welded to each other; and that the welds are located at the vibration antinodes of the ultrasonic wave.

3. Handpiece according to claim 1 or 2,
**characterized in that** at least the probe portion consists of titanium or a titanium compound; and that the diameter of the channel is approximately 2 mm, the thickness of the probe portion is less than 4 mm, and the length is more than 30 cm.

4. Handpiece according to any one of claims 1 to 3,
**characterized in that** the sonotrode has a thickened portion on the proximal side, the thickened portion being welded to the probe portion and screwed to the housing.

5. Handpiece according to any one of claims 1 to 4,
**characterized in that** one single connecting means is provided; and that all handpiece connectors of the connected supply lines are mounted on the one connecting means.

6. Handpiece according to claim 1,
**characterized in that** information from the actuating element is transmitted magnetically, inductively, electro-magnetically, optically and/or acoustically to the switch sensor that is connected to the electrical circuit.

7. Handpiece according to claim 6,
**characterized in that** the actuating element comprises a magnet (3), the magnetic field of which changes at the location of the switch sensor upon an actuation of the actuating element; and
that the switch sensor closes and opens in dependence upon the magnetic field.

8. Handpiece according to claim 6 or 7,
**characterized in that** the switch sensor is a reed relay or a Hall probe.

9. Handpiece according to any one of claims 1 to 7,
**characterized in that** the switch unit permits the properties of at least one electrical circuit to be varied.

10. Handpiece according to claim 8,
**characterized in that** the actuating element(s) have oscillatory circuits of different resonance frequencies; and
that an actuation of the actuating element activates at least one oscillatory circuit at a time.

11. Handpiece according to claim 9 or 10,
**characterized in that** a coil and an electrical circuit are provided in the handpiece for inductively detecting the resonance frequency of the activated oscillatory circuit.

12. Handpiece according to any one of claims 1 to 11,
**characterized in that** only two signal lines are provided in the connected supply line for a switching-on operation and other operations such as the temperature monitoring and the handpiece coding.

13. Handpiece according to any one of claims 1 to 12,
**characterized in that** additionally an HF facility is provided.

14. Handpiece according to claim 13,
**characterized in that** for insulation between high-frequency and ultrasound supply systems in the handpiece, any leakage paths are lengthened or interrupted.

15. Handpiece according to claim 14,
**characterized in that** the insulation is effected at least partially with a PVDF hose and/or an insulating ring.

16. Handpiece according to claim 14 or 15,
**characterized in that** it comprises a housing of a plastics material.

17. Handpiece according to any one of claims 2 to 16,
**characterized in that** the channel in the sonotrode extends linearly through the handpiece as far as a connection for fluid.

18. Handpiece according to claim 17,
**characterized in that** the channel serves for applying suction or rinsing.

19. Handpiece according to claim 18,
**characterized in that** a change-over switch is provided, the actuation of which effects a change-over from an application of suction to rinsing and vice-versa.

20. Handpiece according to any one of claims 1 to 19,
**characterized in that** fastenings, holders or couplings of hoses, tubes, electrical connections and/or seals are mounted closer than λ/8 to vibration nodes.

21. Handpiece according to any one of claims 1 to 20,
**characterized in that** an ablation-promoting element is mounted in the region of the distal (near patient) end of the sonotrode (27).

22. Handpiece according to claim 21,
**characterized in that** the ablation-promoting element is a loop-shaped ring adapted for cutting.

23. Handpiece according to claim 22,
**characterized in that** the mid-perpendicular of the loop-shaped ring adapted for cutting and the axis of symmetry of the sonotrode (27) are disposed to be parallel to each other.

24. Handpiece according to claim 22 or 23,
**characterized in that** the loop-shaped ring adapted for cutting, as seen in at least one projection, is wider at the attachment basis than in the opposite region.

25. Handpiece according to claim 24,
**characterized in that** the conture of the loop-shaped ring is in the shape of an exponential or quadratic curve.

26. Handpiece according to any one of claims 22 to 25,
**characterized in that** the loop-shaped ring is welded to the sonotrode (27).

27. Handpiece according to any one of claims 1 to 26,
**characterized in that** the tip of the sonotrode (27) and/or the cutting blade of the loop-shaped ring consist of, and/or are coated with, sintered carbide.

28. Handpiece according to any one of claims 17 to 27,
**characterized in that** the suction line is used as an instrument or observation channel.

29. Handpiece according to any one of claims 1 to 28,
**characterized in that** high-frequency signal components are coupled out before reaching an ultrasonic generator (28) that provides the supply voltage for the ultrasound facility.

30. Handpiece according claim 29,
**characterized in that** the high-frequency signal components are coupled out by at least one inductive element.

31. Handpiece according to claim 30,
**characterized in that** a compensating coil (22) serves as the inductive element.

32. Handpiece according to claim 31,
**characterized in that** the voltage-carrying cables are wound around the core of the compensating coil (22), and their magnetic fields compensate each other within the core.

33. Handpiece according to any one of claims 30 to 32,
**characterized in that** the voltage-carrying cables are attenuated by capacities to earth.

34. Handpiece according to any one of claims 1 to 33,
**characterized in that** the ultrasonic transducer consists of piezoelectric plates.

35. Handpiece according to claim 34,
**characterized in that** the number of the piezoelectric plates is even; and
that the piezoelectric plates are insulated from the oscillating mass.

36. Handpiece according to claim 34 or 35,
**characterized in that** the generator (21) controlling the ultrasonic transducer has a floating symmetrical output.

## Revendications

1. Pièce à main (25) pour un appareil opératoire endoscopique multifonctionnel, présentant au moins une fonction ultrasonique, comprenant
- un carter,
- des moyens de connexion, disposés audit carter et aptes à être reliés aux conducteurs électriques et également aux conduits de liquides,
- au moins un transducteur d'ultrasons (7),
- une sonotrode ultrasonique (27), qui convoie de l'énergie ultrasonique à une extrémité distale de l'appareil opératoire, et
- au moins un moyen commutateur, disposé sans ligne d'alimentation séparée et des passages de câbles à la pièce à main, présentant un élément de commande et un circuit affecté pour la commande des fonctions de la pièce à main,
**caractérisé en ce**
**que** ledit moyen commutateur comprend un détecteur à commutation, et en ce que ledit élément de commande ne comprend pas un élément apte à être porté en contact mécanique direct avec ledit détecteur à commutation respectif.

2. Pièce à main selon la revendication 1,
**caractérisé en ce que** ladite sonotrode (27) comprend une partie de sonde allongée à un passage composé de plusieurs pièces, qui sont soudées l'une à l'autre, et **en ce que** les points de soudure se trouvent dans les ventres de vibration de l'onde ultrasonore.

3. Pièce à main selon la revendication 1 ou 2,
**caractérisé en ce qu'**au moins la partie de sonde est fait en titane ou en un composé à titane, et **en ce que** le diamètre dudit passage est plus petit que 2 mm environ, l'épaisseur de ladite partie de sonde est moins de 4 mm et la longueur est plus grande que 30 cm.

4. Pièce à main selon une quelconque des revendications 1 à 3,
**caractérisé en ce que** ladite sonotrode présente une partie gonflée du côté proximal, qui est soudée à ladite partie de sonde et vissée audit carter.

5. Pièce à main selon une quelconque des revendications 1 à 4,
**caractérisé en ce qu'**un seul moyen de raccordement est disposé, et
**en ce que** tous les éléments de raccordement à la pièce à main desdites lignes de connexion sont montés audit seul moyen dé raccordement.

6. Pièce à main selon la revendication 1,
**caractérisé en ce que** des informations sont transmis dudit élément de commande par voie magnétique, inductive, électromagnétique, optique et/ou acoustique audit détecteur à commutation, qui est relié audit circuit de commutation.

7. Pièce à main selon la revendication 1,
**caractérisé en ce que** ledit élément de commande comprend un aimant (3) dont le champ magnétique au site dudit détecteur à commutation varie en réponse à la commande dudit élément de commande, et
**en ce que** ledit détecteur à commutation se ferme ou s'ouvre en fonction du champ magnétique.

8. Pièce à main selon la revendication 6 ou 7,
**caractérisé en ce que** ledit détecteur à commutation est un relais du type Reed ou une sonde à effet de Hall.

9. Pièce à main selon une quelconque des revendication 1 à 7,
**caractérisé en ce que** ledit moyen commutateur permet la variation des caractéristiques d'au moins un circuit.

10. Pièce à main selon la revendication 8,
**caractérisé en ce que** ledit ou lesdits élément(s) de commande comprennent des circuits résonnants aux fréquences de résonance différentes, et
**en ce qu'**au moins un circuit résonnant respectif est activé en réponse à la commande dudit élément de commande.

11. Pièce à main selon la revendication 9 ou 10,
**caractérisé en ce qu'**une bobine est un circuit sont disposés dans la pièce à main, qui détectent, par voie inductive, la fréquence de résonance dudit circuit résonnant activé.

12. Pièce à main selon une quelconque des revendications 1 à 11,
**caractérisé en ce que** deux seules lignes d e signalisation sont disposées pour la fonction de mise en circuit, ainsi que des autres fonctions comme la surveillance de température et le codage de la pièce à main.

13. Pièce à main selon une quelconque des revendications 1 à 12,
**caractérisé en ce qu'**au plus un moyen à haute fréquence est disposé.

14. Pièce à main selon la revendication 13,
**caractérisé en ce que** des lignes de fuite éventuels sont prolongés ou interrompus pour l'isolation entre l'alimentation en haute fréquence et l'alimentation en ultrasons dans la pièce à main.

15. Pièce à main selon la revendication 14,
**caractérisé en ce que** l'isolation se fait, au moins en partie, moyennant un tuyau en PVDF et/ou un anneau isolateur.

16. Pièce à main selon la revendication 14 ou 15,
**caractérisé en ce qu'**il comprend un carter en une matière synthétique.

17. Pièce à main selon une quelconque des revendications 2 à 16,
**caractérisé en ce que** ledit passage dans ladite sonotrode s'étend en ligne droite à travers la pièce à main jusqu'à un raccord de fluide.

18. Pièce à main selon la revendication 17,
**caractérisé en ce que** ledit passage sert à l'aspiration ou au rinçage.

19. Pièce à main selon la revendication 18,
**caractérisé en ce qu'**un commutateur est disposé, dont la commande cause la commutation de la fonction d'aspiration à la fonction de rinçage et vice versa.

20. Pièce à main selon une quelconque des revendications 1 à 19,
**caractérisé en ce que** des fixateurs, des éléments de support ou respectivement des coupleurs pour des tuyaux, des conduits et conducteurs, des connecteurs électriques et/ou des éléments d'étanchéité sont montés à une distance de moins de λ/8 des noeuds d'oscillation.

21. Pièce à main selon une quelconque des revendications 1 à 20,
**caractérisé en ce qu'**un élément amplifiant l'érosion est disposé dans la région de l'extrémité distale (proche au patient) de ladite sonotrode (27).

22. Pièce à main selon la revendication 21,
**caractérisé en ce que** ledit élément amplifiant l'érosion est un anneau en boucle coupant.

23. Pièce à main selon la revendication 22,
**caractérisé en ce que** la médiatrice dudit anneau en boucle coupant et l'axe de symétrie de ladite sonotrode sont disposés l'un en parallèle à l'autre.

24. Pièce à main selon la revendication 22 ou 23,
**caractérisé en ce qu'**en au moins une projection, ledit anneau en boucle coupant est plus large à la base de fixage que dans la zone opposée.

25. Pièce à main selon la revendication 24,
**caractérisé en ce que** le contour dudit anneau en boucle suit une courbe exponentielle ou carrée.

26. Pièce à main selon une quelconque des revendications 22 à 25,
**caractérisé en ce que** ledit anneau en boucle est soudé à ladite sonotrode (27).

27. Pièce à main selon une quelconque des revendications 1 à 26,
**caractérisé en ce que** la pointe de ladite sonotrode (27) et/ou la lame dudit anneau en boucle sont faits en métal dur et/ou sont recouvert.

28. Pièce à main selon une quelconque des revendications 17 à 27,
**caractérisé en ce que** le conduit d'aspiration est utilisé en tant que passage d'instrument et/ou d'observation.

29. Pièce à main selon une quelconque des revendications 1 à 28,
**caractérisé en ce que** des fractions de signaux à haute fréquence sont sorties devant un générateur d'ultrasons (28), qui fournit l'alimentation de tension pour la fonction ultrasonique.

30. Pièce à main selon la revendication 29,
**caractérisé en ce que** lesdites fractions des signaux à haute fréquence sont sorties moyennant au moins un élément inductif.

31. Pièce à main selon la revendication 30,
**caractérisé en ce qu'**une bobine de compensation (22) sert en tant qu'un élément inductif.

32. Pièce à main selon la revendication 31,
**caractérisé en ce que** les câbles sous tension sont enroulés autour le noyau de ladite bobine de compensation (22) et **en ce que** leurs champs magnétiques se compensent dans le noyau.

33. Pièce à main selon une quelconque des revendications 30 à 32,
**caractérisé en ce que** les câbles sous tension sont amortis par rapport à la terre moyennant des éléments capacitifs.

34. Pièce à main selon une quelconque des revendications 1 à 33,
**caractérisé en ce que** ledit transducteur d'ultrasons est composé des disques piézo-électriques.

35. Pièce à main selon la revendication 34,
**caractérisé en ce que** lesdits disques piézo-électriques sont isolés de la masse en rotation.

36. Pièce à main selon la revendication 34 ou 35,
**caractérisé en ce que** ledit générateur (21), qui commande ledit transducteur d'ultrasons, comprend une sortie symétrique mobile.
